# EUROPEAN PATENT APPLICATION

(11) **EP 3 895 759 A1**
(43) Date of publication of application: **20.10.2021**
(21) Application number: 21168380.0
(22) Date of filing: 14.04.2021
(51) Int. Cl.: A61N 1/36, A61N 1/04, A61B 5/024, A61B 5/053

(54) **GARMENT AND METHOD FOR TRANING MUSCLES**

(30) Priority: 14.04.2020 CH 4442020
(71) Applicant: MikeFit GmbH, 6340 Baar (CH)
(72) Inventor: Lussambo, Michael, 6340 Baar (CH)
(74) Representative: IPrime Rentsch Kaelin AG

(57) **Abstract**

Disclosed are a garment and a method for training muscles of a user wearing the garment. The garment comprises sensors which are embedded into the garment for electrical contact with a skin surface of the user when the garment is worn. Activators are embedded into the garment to stimulate one or more muscles. A controlling unit is attached to the garment for receives and processes signals representing an electrical impedance of one or more muscles. The stimulation of the one or more muscles through the activators is generated only when one or more muscles are in an un-exhausted state.

## Description

The present invention relates to a garment and a method for training muscles. In particular, a wearable body suit is disclosed for use in the medical and sports area and particularly for effective physical workouts.

### Background of the Invention

Technical wearables are known including devices for the stimulation of muscles.

WO 2016/131936 A2 relates to a device, system and method for transmitting stimuli to a user. Said stimuli can comprise stimuli caused by electrical muscle stimulation or haptic stimuli such as vibrations. The system simplifies the use of the corresponding stimuli inter alia in that parameters can be measured during the use and the type and specificity of the stimuli can be changed depending on the measured parameters.

WO 2016/149751 A1 relates to muscle activity monitoring and particularly to a system for monitoring muscle activity of a biological subject. The system includes at least one garment including a number of arrays of electrodes positioned on the garment so that when the garment is worn by a subject in use, the electrodes contact skin of the subject and generate electrical signals indicative of electrical potentials within respective muscles of the subject and at least one electronic processing device that processes signals from the electrodes in each electrode array to determine a muscle activation for parts of the respective muscles and uses the muscle activation to determine at least one muscle indicator indicative of muscle activity of the subject.

US 2015/366504 A1 relates to electromyographic clothing. An article of clothing with electromyographic (EMG) sensors measures body motion and/or muscle activity. This clothing can be a short-sleeve shirt or a pair of shorts, wherein the electromyographic (EMG) sensors are on the cuffs. The electromyographic (EMG) sensors can be modular; they can be removably attached to different locations in order to create a customized article of electromyographic clothing which optimally measures the muscle activity of a particular person or muscle activity during a particular sport. This clothing can also include bending-based motion sensors.

There is a general need for advancements in this field of technology.

### Summary of the Invention

It is an object of the invention to provide a garment for training muscles of a user in less time than normal training while considering the health of a muscle or groups of muscles. As time and health are precious resources it is key to maintain physical health with an effective training. A wearable body suit should improve training sequences and provide information about health.

It is another object of the invention to provide a method for training muscles of a user wearing a garment.

These and other objects are achieved by an inventive garment and an inventive method according to the independent claim. Other preferred embodiments are indicated in the dependent claims.

A first aspect of the invention relates to a garment for training muscles of a user. The garment comprises sensors which are embedded into the garment for electrical contact with a skin surface of the user when the garment is worn. Activators are embedded into the garment to stimulate one or more muscles. A controlling unit receives and processes signals representing an electrical impedance of one or more muscles. The stimulation of the one or more muscles through the activators is generated only when one or more muscles are in an un-exhausted state. Generally, a muscle is in an exhausted or fatigue state when an increase in the reflective part in coping with a strain load is determined or detected. The controlling unit determines the transition from an un-exhausted to an exhausted state and thus can control and stop the stimulation for one or more muscles.

The sensors and the controlling unit are adapted to determine the electrical impedance representing a level of muscle fatigue by one or more of electrical impedance myography (EIM), electromyography (EMG), and a body composition with a process of bioelectrical impedance analysis (BIA).

The activators can comprise electrodes which are adapted to stimulate the one or more muscles by electronic muscle stimulation (EMS). Standard electrodes can stimulate muscles, but they cannot be used to measure muscle voltage, thus specific electrodes are used for both stimulation and measurement.

The sensors and the activators can advantageously be implemented as electrodes or pads which are distributed over the garment and arranged according to respective muscles or muscles groups. In a preferred embodiment approximately 20 individual muscles are controllable by around 40 electrodes or pads. In general, an electrode or pad can have the function of a sensor as well as activator or stimulator.

The sensors, activators, and connect via wires to the controlling unit. If the sensors and activators, i.e. the electrodes are laminated onto the garment good electrical contact with a user's skin surface can be achieved.

The garment as a wearable training or body suit allows to optimize an electronic muscle stimulation (EMS) while considering preferably at the same time electrical impedance myography (EIM), electromyography (EMG) and bioelectrical impedance analysis (BIA). In a preferred embodiment all analyses or methods are applied. Other combinations like EMS with EIM and BIA, or EMS with EMG and BIA are possible. EMS can be used to stimulate the muscles which increases the efficiency of the training. The EMG measures the muscle's voltage which can be used to detect the muscle's condition. BIA is used to determine the composition of the human body e.g. body fat, total body water and more. The garment with all parts can be considered to be an EMS system that includes at least two measurement systems, like EMG and BIA.

When activators stimulate the one or more muscles by a controlled current up to 30 mA then the muscles can be stimulated application dependent in an optimized way allowing for more efficient and time-saving exercises.

Muscle voltages can be measured or determined which can reach a maximum of ±5 mV with EMG and can additionally perform a BIA. One EMS impulse sends a limited amount of current through a muscle and forces a contraction that does not affect movement but does exhaust the muscle. With the measured EIM and/or EMG the movement and contraction of a muscle can be traced providing information about the condition of the muscle. Therefore, every muscle can be trained individually, but is only trained as long as it is not exhausted or fatigue. The level of exhaustion or fatigueless can also be determined by a shift in the frequency components of the EMG towards lower frequencies. The BIA provides information about a user's body condition and allows the subject keep track of a muscle's progress over time. With BIA an impedance can be determined by measuring a resulting voltage. With the impedance many health indicating values can be calculated such as lean body mass, body fat, total body water and more can be extrapolated. With a combination of EMS, EIM, EMG and BIA a training can be designed to optimally train a muscle without damaging it. Such a training suit can be extended with more functions such as a pulse measurement, step counter, gyro sensor, acceleration sensor and more.

The controlling unit can be attached to the garment. This is particularly advantageous without any cables. The controlling unit can control the muscle stimulation, record measurement data, and can communicate with a mobile application at an external device via, e.g. Bluetooth® Low Energy. The mobile application, also referred to as APP, can control the garment as a workout suit, illustrate measurement data, control the stimulation intensity, and communicates via Bluetooth® Low Energy with the controlling unit. Data records can be stored locally, but also in a distributed system. The controlling unit can receive signals which represent an electrical impedance of one or more muscles. The signals can be processed to determine the electrical impedance which is a measurement of said impedance. Based on the measurement or in response to the processed signals the one or more muscles can be stimulated when one or more muscles are in an un-exhausted state. That means if a muscle is fatigue then no stimulation is applied. By doing so no damage to the muscle can occur and the particular non-stimulated muscle can relax for a longer period.

The controlling unit can comprise a microcontroller that controls the signals to and from the electrodes or pads. The controlling unit can further control the communication with a mobile application.

If the controlling unit can connect to an external sensor, such as one of a smart watch worn during exercise by a user, additional useful input can be considered and processed for an optimized training effects.

The garment may comprise signaling means for generating feedback. For example, a vibration occurs or a sequence when an exercise is not executed correctly, or at certain time intervals or when a training session is over. The same can be achieved by an acoustic signal communicating, e.g. the beginning and end of an exercise. Further, a voice output can communicate a correction of the form within an instructed movement. This allows for real time feedback for movement control. For example, if a movement is too fast, an indication can be given to perform the movement slower.

Another aspect of the invention relates to a method for training muscles of a user wearing a garment. The method comprises the steps of receiving signals representing an electrical impedance of one or more muscles; processing the signals to determine the electrical impedance; and in response to the processed signals stimulating the one or more muscles when one or more muscles are in an un-exhausted or non-fatigue state. The method may comprise the step of determining or detecting an exhausted or fatigue state or the beginning of the said state when an increase in the reflective part in coping with a strain load happens.

The step of processing the signals may further comprises determining the electrical impedance representing a level of muscle fatigue by one or more of electrical impedance myography (EIM), electromyography (EMG), and a body composition with a process of bioelectrical impedance analysis (BIA).

It is particularly advantageous if the measuring and stimulation of one or more muscles are performed substantially at the same time or synchronously, because then the best effects can be achieved.

If signals can be received from an external sensor, further data can be considered and processed. Such signals might be indicative of acceleration of a body or body parts, heart rate frequence, heart rate variability pulse, body-core temperature, and/or body hydration.

In an embodiment a non-fatigued muscle potential is determined. In a further embodiment a stimulation stop or refusal to start due to muscles that are too tired or insufficiently regenerated is determined. In yet another embodiment an automated selection of the continuously changing optimal size of the muscle stimulation is applied. An automatic exercise selection can be provided through a self-leaning system and smart gesture intensity design/ & audio real-time feedback.

Overall, an optimization of all free and guided movements by the self-learning system or with a trainer can be achieved. It is also advantageous that a reduction of the risk of injury in planned and unscheduled movements can be achieved.

### Brief Description of the Drawings

In order to facilitate better understanding of the present invention, reference is made below to the drawings. These show only exemplary embodiments of the subject matter of the invention.

In the figures and the associated description, identical or functionally analogous parts are provided with the same reference numerals.
Fig. 1 shows a shape of a garment for training indicating certain areas and a controlling unit.
Fig. 2 shows a garment with an external device and an external sensor.
Fig. 3 shows a flow diagram for training muscles of a user while wearing the garment.
Fig. 4 shows a block diagram of the components for use with a garment and training muscles of a user.
Fig. 5 shows a schematic diagram with various modules of the controlling unit.
Fig. 6 shows a schematic training set up with camera and display.
Fig. 7 shows a further schematic training set up with camera and display.

### Detailed Description

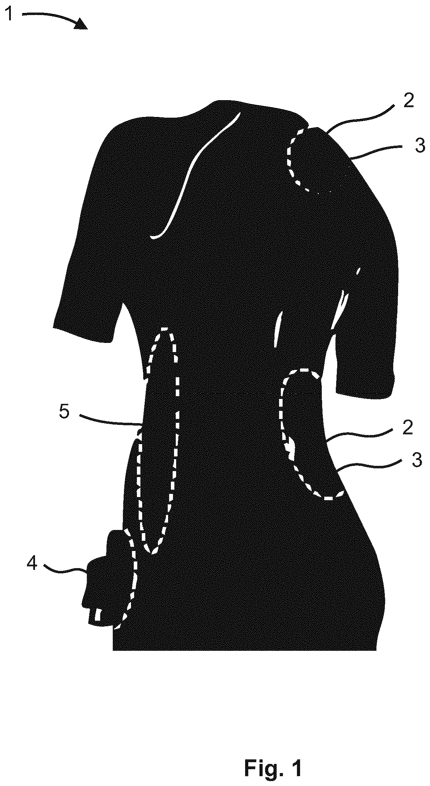

**Figure 1** shows a silhouette of a garment 1 for training muscles of a user. The garment or training suit 1 comprise sensors 2 and activators 3 in certain areas of the garment 1 (only two areas are indicated). The sensors 2 and activators 3 are implemented as electrodes or pads 2, 3 as indicated for two areas in the figure. The electrodes 2, 3 are arranged or located at muscles or muscle groups to be measured, stimulated, and trained.

The sensors 2 are embedded into the garment 1 for electrical contact with a skin surface of the user when the garment is worn. In a preferred embodiment ca. 20 individual muscles are controllable by around 40 electrodes or pads 2, 3. The training suit 1 fits tightly for a good electrical contact with a user. The activators 3 are embedded into or laminated onto the garment to stimulate one or more muscles. A controlling unit 4 is arranged at the lower part of the garment 1. In a further embodiment the controlling unit 4 is placed in the area of the chest, preferable in the middle. The controlling unit 4 receives and processes signals that represent an electrical impedance of one or more muscles. The sensors 2 and activators 3, i.e. the electrodes or pads 2, 3 are connected via wires 5. The wires 5 can be silver wires or special wires woven into or onto the material of the garment 1. The garment 1 consists of a high-tech fabric that is light and washable and comes with various other advantageous features. The wires 5 are characterized by longevity, are washable at least a few times, have a high conductivity, do not need much space, and are stretchable with a high flexibility. Nanoleq products such as StretchOne are stretchable electronics that can be applied. In general, the electrodes and conducting paths can be provided by printing and laminate techniques.

A stimulation of the one or more muscles through the activators 3 or electrodes is generated when one or more muscles are in an un-exhausted state. The term muscle refers to skeletal muscles. Known stimulation clothing or devices stimulate continuously. If such a muscle is exhausted or in a fatigue state, then no stimulation is applied. This protects the muscles, has a maximized training effect, and strengthen the muscle without overdoing. Thus, it is relevant to determine the state of the muscles.

A muscle contraction usually stops once the nervous system stops signaling the muscle or if the muscle runs out of adenosintriphosphat, which is the energy source of the cells. However, as the garment 1 with the electrodes 2, 3 and the controlling unit 4 provides measurement and stimulation of the muscles or groups of muscles it is determined whether a muscle is in or turns in an exhausted or fatigue state. The latter is the case when an increase in the reflective part in coping with a strain load is determined.

Generally, muscle stimulation can be used to evaluate the level of health and provide data about the human body.

With electrical muscle stimulation (EMS) muscles are stimulated by an electrical signal. Normally, to contract a muscle the brain sends a very small electrical signal to the muscle. Thus, it is possible to stimulate the muscle with an outside electrical signal without the brain sending one on its own. The signal to contract the muscle which is sent from the outside of the body has to flow through a point of the skin. This can be achieved by placing two electrical conducting electrodes or pads 3 as activators on the skin over the muscle. The controlling unit 4 connected to these pads 3 generates a current which, depending on the application can be up to 30 mA, 50 mA or 100 mA, whereas up to 30 mA is preferred. The current can have different shapes. It can be either unidirectional or bidirectional. Usually it consists of short pulses with long pauses in between a pulse. Numerically these pulses are about 180-200µs and come at an interval of about 50 or 100Hz. This means the pause is about 10-20ms. In the case of a bidirectional pulse, sometimes there is an additionally pause between the positive and the negative pulse that is also referred to as interpulse interval. This pause is about 0-250 µs long. Depending on the impedance of the stimulated muscle the current flowing through the muscle results in a different voltage. The resistance of a muscle makes up approximately 90% of the muscle and has in average a value of 1 kOhm to 1.5 kOhm per muscle.

While using EMS, a person's muscles can be forced to make mini contractions during a training session. EMS is used by garment 1 to stimulate the muscles during an exercise to make the muscles work harder. As a result, a workout can take less time but have the same outcome. Making exercises and workouts more efficient.

In electrical impedance myography (EIM), high-frequency alternating electrical current is applied to localized areas of muscle via surface electrodes 2 and the consequent surface voltage patterns are analyzed. In contrast to conventional electromyography (EMG), EIM does not focus on measuring the inherent electrical activity of the tissues. Rather, similar to diagnostic ultrasound, measurements are made over a small area of interest, with energy being applied to the body and the resultant surface patterns analyzed. In EIM, electrical current is used, and the output is a set of quantitative parameters describing the state of the muscle.

Electromyography (EMG) allows to measure the voltage a muscle generates while contracting so as to record the electrical activity of muscle tissue. That means, naturally occurring electrical voltages within a muscle are measured.

The functional principle behind an EMG is the voltage, which is -80 mV to -90 mV at the fiber membrane of a relaxed muscle and increases to 30 mV when the muscle is contracted. Since the measurement is made on the skin, it can be only measured within a range of ±5000 µV. This voltage is smaller than the voltage at a fiber membrane because the signal has to go through the muscle tissues, fat tissue and through the skin. The measurement of a raw EMG signal is not easy because of this small voltage range and, as a result, interfering signals have an impact. Generally, three electrodes or pads, such as sensors 2 are necessary for a measurement: two over an active muscle and a third one on a much less active muscle. The third pad sets the potential level of the human body to the same level as an amplifiers output. This is necessary because a measurement against a different potential could cause the measured potential to not be in the range of the amplifier, preventing its amplification. The other two pads are used to measure the muscle voltage differentially.

EMG allows to discover what the muscles are doing. This measurement shows how the muscle works and the quality of their work. The muscle function can be detected, and any abnormality recognized.

Bioelectrical impedance analysis (BIA) provides a relatively exact indicator about the physical health of a person. BIA determines the body fat in relation to the lean body mass. The controlling unit 4 sends via the sensors 2 a small, harmless alternating current through the body of the user wearing the garment 1. The resulting voltage over the body is measured and with the results, the lean body mass is determined.

In order to measure the BIA, two electrodes, such as the sensors 2 and/or activators 3, are connected to the human body skin of the user wearing the garment 1. The controlling unit 4 connected to the electrodes 2, 3 generates an alternating current of up to 800 µA and measures the voltage resulting over the body. With the measured voltage and the set current the controlling unit 4 determines a phase angle and an impedance. With these values the reactance and the resistance are determined. With additional processing the controlling unit 4 then evaluates the total body water and with this the lean body mass, as well as the amount of body fat as different body parameters. These values provide a picture of someone's physical health. Further the values can be used to determine the muscle mass and the water distribution in the extra- and intracellular spaces.

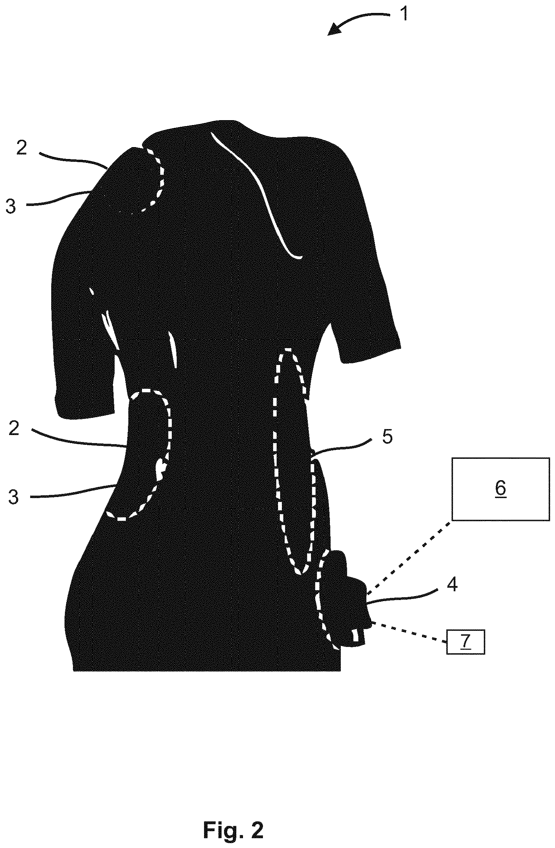

Turning now to Figure 2 which shows a garment 1 with an external device 6 and an external sensor 7. The external device 6 as well as the external sensor 7 can connect wirelessly to the controlling unit 4, e.g. via Bluetooth® Low Energy. The external sensor 7 can be a smart watch or the like that a user wears during an exercise at a wrist. The external sensor 7 provides useful additional information, like pule or acceleration to the controlling unit 4. The external device 6 runs an application, short APP, to control the garment 1, illustrate measurement data, control the stimulation intensity, and to communicate with the controlling unit 4.

In an embodiment the garment 1 can be a T-Shirt or the like with at least two activators 3 creating defibrillation if necessary. The pulse and/or other parameters are detected by the external sensor 7. Based on the processed parameters a defibrillation is created for e.g. resuscitation. The administration of electric shocks may help persons in order to reset normal heart rhythm. The activators 3 are arranged accordingly.

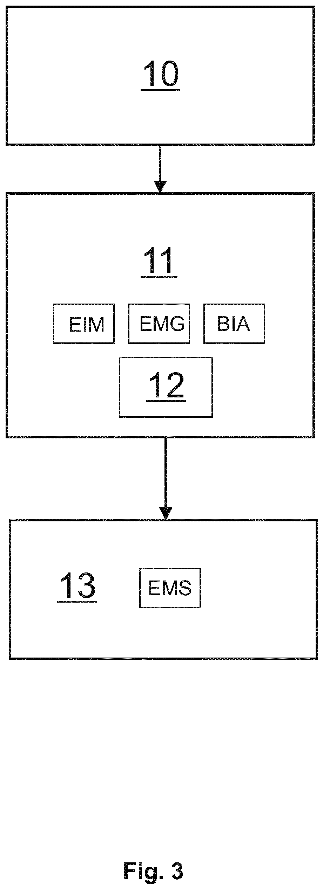

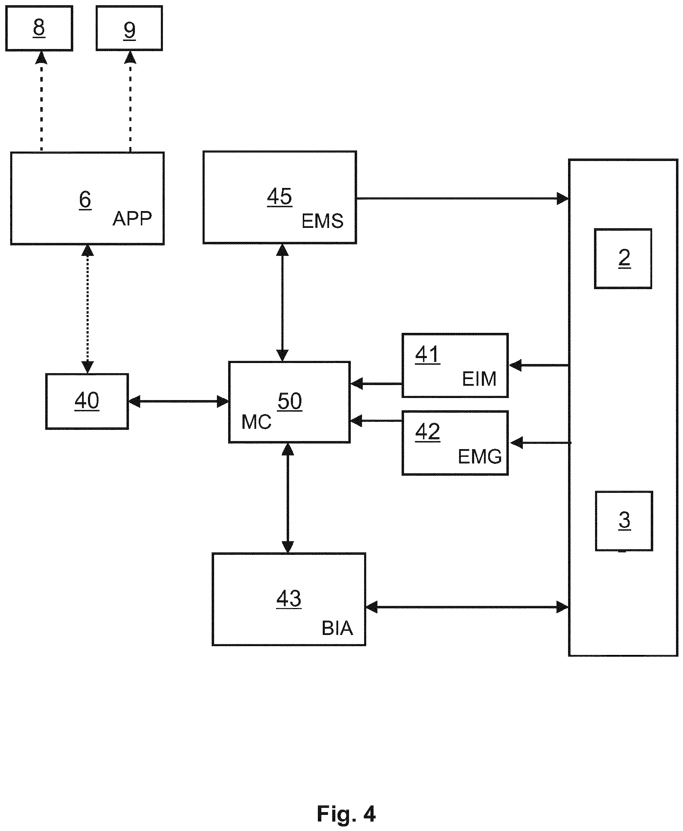

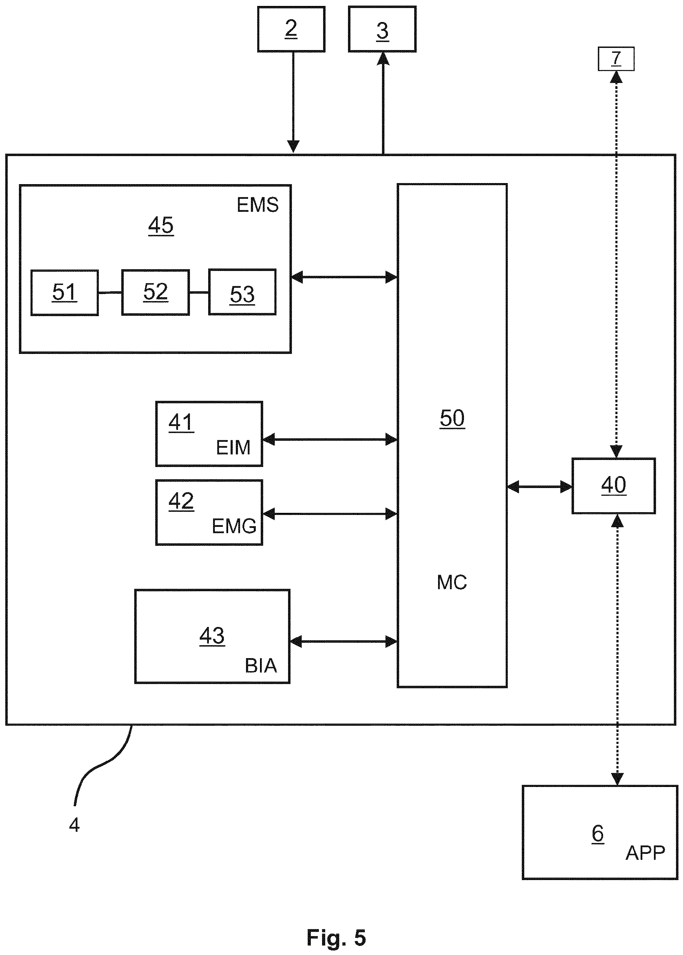

Figure 3 shows a flow diagram for training muscles of a user while wearing the garment 1. In step 10 signals representing an electrical impedance of one or more muscles of a user are received. Next, in step 11 the signals are processed to determine the electrical impedance. The electrical impedance representing a muscle's level of fatigue or exhaustion is determined by electrical impedance myography (EIM), electromyography (EMG), and a body composition with a process of bioelectrical impedance analysis (BIA). In step 12 is determined whether the one or more muscles are in an exhausted state. In step 13, in response to the processed signals the one or more muscles are stimulated when one or more muscles are in an un-exhausted state. No stimulation takes place, e.g. when one or more muscles are fatigue. In other words, the stimulation to one or more muscles is performed as long as the one or more muscles are in an un-exhausted state. This state is determined by step 12 by the controlling unit 4. EIM, EMG, BIA and the EMS are combined to provide an optimized muscle stimulation. All are connected to a microcontroller 50 (show in Figs. 4 and 5) of the controlling unit 4 which controls the communication with the mobile application and potentially the external sensor. The application supports the saving of data records which can be locally, but also in a distributed system.

While responding to the commands of the mobile application the controlling unit 4 starts and controls the EMS and the EIM, EMG, BIA. All the measured data is sent to the mobile application of the external device 6. The user interface of the mobile application provides the relevant buttons and plots to control the controlling unit 4 and shows the results of the measured data.

Figure 4 shows a block diagram of the components for use with garment 1 in order to train muscles of a user effectively. Shown is an external device 6 that is connected via a wireless module 40, e.g. via Bluetooth®, to a microcontroller 50 within the controlling unit 4. The external device 6 can be a tablet or any other computing device running a mobile application, short APP. For example, via Bluetooth® Low Energy the mobile application communicates with the microcontroller 50 and controls the controlling unit 4. The external device 6 provides signaling means for generating feedback to an exercise a user is performing. An acoustic signal 8 can indicate beginning and end of an exercise, but also a sequence of tones may indicate to perform the exercise slower of faster, respectively. In a further embodiment, the feedback is provided by a voice output 9 communicating a correction of the form within an instructed movement. The controlling unit 4 comprises four modules: an EIM module 41, an EMG module 42, A BIA module 43, and an EMS module 45. The modules 41 to 45 provide the following functions: electrical impedance myography (EIM), electromyography (EMG), bioelectrical impedance analysis (BIA), and electrical muscle stimulation (EMS), respectively. The sensors 2 and activators 3 are connected accordingly. EMS is an output only since it does stimulate the muscles. Whereas EIM, EMG is input which measures the voltage within a muscle when it is not stimulated. The BIA acts as an input and as an output. It stimulates and measures at the same time.

Figure 5 shows various schematic modules of the controlling unit 4 that is connected to electrodes, i.e. sensors 2 and activators 3. The controlling unit 4 is further connected to an external device 6 that runs one or more applications, e.g. APPs. The connection is established via a wireless module 40, e.g. via a Bluetooth® module. During the stimulation and measurement the mobile application has the ability to adjust the intensity of the muscles and to stop the process with a command "stop". If at any point of the usage the Bluetooth® Low Energy connection should shut down the EMS module 45 and the EIM, EMG and BIA modules will be stopped immediately. The wireless module 40 can also be connected to an external sensor 7 for receiving additional data. The controlling unit 4 comprises various modules providing EIM, EMS, EMG, and BIA, all are connected to a microcontroller 50. Not all parts or components of the circuity of the modules are shown or explained in detail as these are apparent to those skilled in the art.

In an embodiment, a firmware running on the controlling unit 4 is controlled by the mobile application. It executes the EMS stimulation and measures the EIM, EMG and BIA values. The intensity values for every muscle are received which allows to start and stop the EMS and EIM, EMG sequences. The data can be saved locally for further processing.

The microcontroller 50 reads the measured values of the EIM, EMG and the BIA and controls the stimulation vis the EMS. The microcontroller 50 has its power supply of 5 volts from a constant voltage circuit (not shown). The constant 5 V power supply regulates the power supply of 7.4 V down to 5 V using a linear regulator. The 5 V are used as a power supply for an operational amplifier of the EMG module 42 and as an input voltage for a DC/DC-converter. In an embodiment five signals are used to control the EMS module 45, whereas four signals steer four optocouplers and one the analogue voltage that regulates the current control part. The EMG module 42 is usually only read. The output of the measurement circuit of the EMG module 42 is connected to a general-purpose input/output (GPIO) of the microcontroller 50 with an analogue-to-digital converter function. The same condition applies to the BIA module 42 which also is connected to a GPIO with similar characteristics.

The EMS module 45 comprises a stimulation power supply 51, a current control 52, and a leading sign changer 53.

The stimulation power supply 51 generates at least 60 volts from a 5 volt. In order to generate 60 volts from 5 volts a circuit comprises a DC/DC-converter whereby the resistors set the ratio for the converter to generate the 60 volts.

In general, the electric muscle stimulation comprises two parts: the impulse signal and the current control. The pulse should thus be limited by controlling the current.

Since the EMS module 45 generates current pulses, the amplitudes of these pulses have to be regulated. This is achieved by the current control 52. Within this current control 52 an operational amplifier receives an analogue signal, from the microcontroller 50 and commands a transistor to set the voltage over a current limiting resistor to the same value as the received analogue signal.

In order to prevent blood poisoning the current flowing through a muscle needs to flow in both directions at the same level which makes the leading sign changer 53 relevant. This changer 53 is connected to the current control 52 via H-bridge+ and H-bridge- and to the muscles through a multiplexer (not shown) via signals. The current flows from H-bridge+ to H-bridge- and its direction through the muscles can be controlled by optocouplers (not shown). The current flows through the muscles in one direction and by switching circuity the current flows in the opposite direction. Thus, as seen from the muscle, the current changes its direction of flow.

In a preferred embodiment the EMS module 45 comprises two 32 channel analogue switches and two multiplexers. The analogue switches connect the leading sign changer 53 with all e.g. 20 muscles to be addressed. The switches can be controlled directly by the microcontroller 50. A resistor can be added to the 21st channel which can be considered to be an additional muscle like a fake muscle. When the stimulation impulse is generated with the leading sign changer 53 the edges of the stimulation impulses may not reach the desired voltage as fast as when generated with an analogue switch, i.e. have a different starting characteristic. The multiplexers fulfill the same function, they connect every muscle with the EMG module 42. They handle very small signals and do not tolerate much voltage. To protect them from too large external voltages they have a protection circuit consisting preferably of two resistors and two diodes whereas one of the diodes is bidirectional. This bidirectional diode protects the input of the multiplexer from electrostatic discharge and limits the voltage to ±60 volts. If this limit were lower, the diodes would interfere with the EMS signals. The resistor of 120 kOhm and the Z-diode protect the multiplexer from the remaining 60 volts by forming a voltage divider. The voltage on the input of a multiplexer cannot be any higher than 3.3 volts forced by the Z-diode. In case of a negative pulse of an electrostatic discharge, the Z-diode cannot protect the multiplexer anymore, therefore, a current limiting resistor of 120 Ohm should be added.

Turning to a further embodiment. In order to produce an impulse which stimulates a muscle analogue switches connect the stimulation power supply 51 to the muscle. Additionally, the leading sign changer 53 connects the current control 52 through to the analogue switches. This means that the analogue switches and the leading sign changer 53 form a serial connection of two switches. Both of these switches can be used to generate the EMS pulses. Because these two switches are different components a difference in the rising edge can be noticed. The rising edge differs whether the analogue switch or the leading sign changer 53 closes the circuit. Using the leading sign changer 53 to generate the impulse the rising flank is slightly different as when analogue switches are used. Thus, analogue switches are preferred as there is a difference between the two switching methods. The difference of the impulse is a small peak before every rising edge and within the edge itself which does not rise fast enough.

The EMS module 45 stimulates muscles, each stimulated muscle makes a contraction and the EIM module 41 and/or EMG module 42 measures the muscle voltage. Within this sequence it is possible that the muscle cannot recover fast enough from the stimulation and is still in contraction. The EIM module 41 and/or EMG module 42 then measures said contraction but detects a much higher value than with a purposely made contraction because the EMS stimulates the muscle stronger than the body would when contracting.

The EIM module 41 creates a high frequency alternating electrical current that is applied to localized areas of muscles. The resulting surface voltage patterns are processed and analyzed by the controlling unit 4.

The EMG module 42 is also for measurement. The muscle voltage is in a range of ±5 mV. In order to measure a muscle voltage a differential operational amplifier is applied. The signal difference is measured twice, making the measurement exacter. Alternatively, two amplifiers in one can be used. Muscle voltage has a bandwidth of 6 Hz to 600 Hz. Therefore, there are two low pass filters, whereby one operates through the feedback loop as a high pass filter. In an embodiment, the cutoff frequency at the -3 dB point is set to 1.2 kHz because it should allow the 600 Hz signals to pass through it and not dampen it. For the low pass filter the cutoff frequency is set to 3 Hz so as to not dampen the 6 Hz signals.

The EMG module 42 provides data as long as the EMS has not been started. This means as long as the stimulation intensity is set to 0% (equals 0 mA) the measurement will show the contraction of a muscle. As soon as the stimulation of a muscle is set to a value between 1 and 100% the signal of the EMG module 42 rises to a value of about 3.3 V. The microcontroller 50 reaches the limitation of its measurement range and returns the maximal possible value and no further contractions are visible within the measured signal. This is because the EMS would first contract the muscle and the charge would settle down. Afterwards, the EMG would measure the still contracted muscle and provide a much higher value than expected, since the contraction would not be forced by the strength of the body, but by stimulation. Therefore, the contraction generated by the EMS would be stronger than any contraction made by the body itself.

Generally, the EMG module 42 is able to measure a muscle's contraction. The two 32 channel multiplexers connect the two inputs of the EMG module 42 with the wanted muscle. If for example the channel number 1 has no connection to a muscle, perhaps due to a bad fitting of the garment 1, this input is on a level of 0 V. Afterwards the channel 2 becomes connected for a measurement which itself is at a voltage level of 1.65 V in addition to the measurement signal. The connection of the second channel then lifts the input of the measurement circuit from 0 to 1.65 V.

As indicated, the intensity of a stimulation depends on different factors, i.e. on both: the duration of a pulse and the interpulse interval (IPI), i.e. the pause between a positive and a negative stimulation pulse, have an impact on the result. The positive as well as the negative pulse have a duration of 180 µs and the IPI can be set to 250 µs. In the firmware, these times are set with hardware interrupt timers which only set a flag to indicate that the stimulation can start and what will be executed in a main loop. After each muscle has been stimulated with one positive and one negative pulse an EMG measurement is performed once for each muscle. For this measurement the firmware tells the multiplexers to connect the first muscle with the EMG module 42. Afterwards the signal settles for approximately 400 µs before it is measured. The firmware then connects the EMG module 42 with the second muscle and lets the signal settle again. This cycle is repeated for each muscle. Directly after the EMG measurement, values are sent via Bluetooth® Low Energy to the mobile application. The stimulation, the EMG and the sending take approximately 20 ms. Therefore, this whole process will be repeated with a frequency of 50 Hz (20 ms). The stimulation itself is intermeshed. Since the IPI of a muscle is about 250 µs the whole impulse of 180 µs can fit in this IPI. Therefore, two muscles are stimulated nearly at the same time. A positive pulse on muscle one is followed by a positive pulse on muscle two before the negative pulse on muscle one is generated. The positive pulse of muscle two is intermeshed into the IPI between the positive and the negative pulse of muscle one.

The BIA module 43 comprises in an embodiment five elements, four resistors and a differential amplifier. The resistors work as two voltage dividers as they break the measurement signal down for the differential amplifier which amplifies the measured difference. The output of the amplifier is connected to the microcontroller 50.

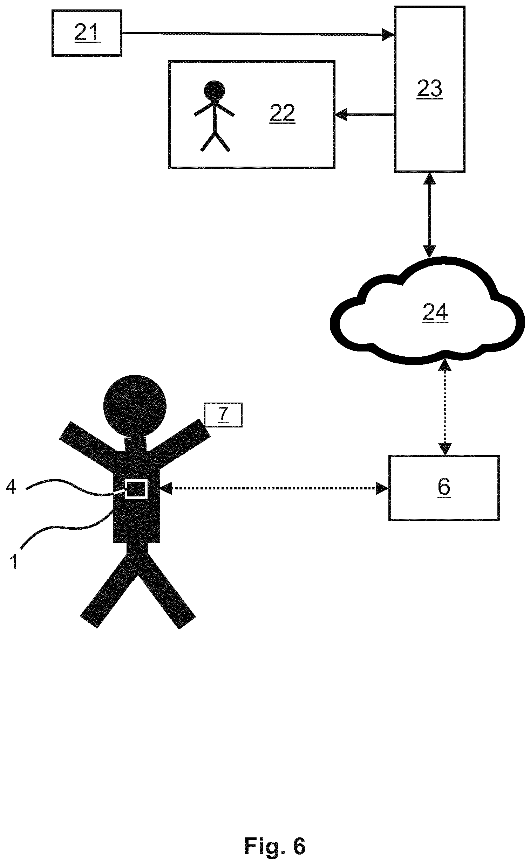

Figure 6 shows a training set up with a camera 21 and a display 22 for movement control and correction. The electronic or video camera 21 and the display 22 are connected to a processing unit 23, such a Raspberry Pi, that is further connected to the Internet 24 with cloud services for processing and storing data.

A user wearing a garment or training suit 1 is in front of the camera 21 and can see or receives information from the display 22, such as a monitor. The training suit 1 has a controlling unit 4 arranged in this embodiment in the chest area of the training suit 1. The user wears an external sensor 7, such as a smart watch, that connects either to the controlling unit 4 and/or an external device 6. The external device 6, can be a tablet that further connects to the Internet 24 and thus has access to the cloud services and storage. In a further embodiment the external device 6 is directly connected to the processing unit 23.

The movements of the user as seen or recorded by the camera 21 are processed by the processing unit 23 and in combination with the data from the processing unit 4 and the external sensor 7 to the external device 6, movements are visualized to guide the user with real time feedback during an exercise. A moment analysis is performed either by the processing unit 23 and/or the cloud services. The feedback for corrections or improvements is shown to the user via the display 22.

A real-time visual motion analysis is performed and combined with a feedback loop. This can be based on a pose detection and allows to derive and provide information about joint angle (posture & imbalances), number of repetitions, exercise duration, ground contact times. With this information, training efficiency can be increased while reducing the risk of injury. The pose detection detects the pose of a subject's body in real time from a continuous video or static image. A pose describes the body's position at one moment in time with a set of skeletal landmark points including facial landmarks such as ears, eyes, mouth, and nose and also points on the hands and feet. The landmarks correspond to different body parts such as the shoulders and hips. The relative positions of landmarks can be used to distinguish one pose from another.

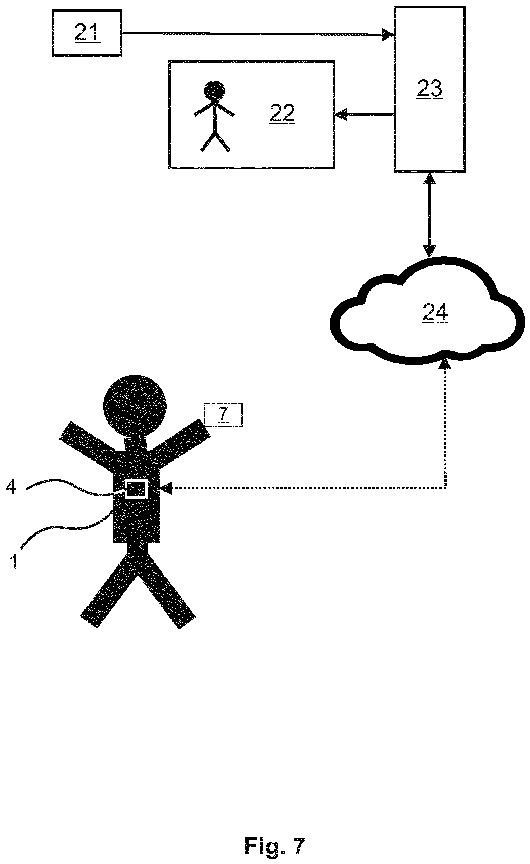

Figure 7 shows a further training set up with a camera 21 and a display 22 for movement control and correction, but without an external device 6. In this embodiment, the controlling unit 4 is directly connected to the Internet 24. For that the controlling unit 4 has WLAN functionalities. In a further embodiment, when the controlling unit 4 uses Bluetooth®, a further device (not show) connects to the Internet 24.

When the controlling unit 4 has no connection with the Internet 24 and/or an external device 6 a self-sufficient function can be provided, and the stimulation is continued. The garment 1 with the controlling unit 4 is then in an autarkical mode or function. Whenever the connection to the external device 6 and/or the Internet 24 is given or re-established, all other described functions are available.

Although the subject matter has been described in terms of certain embodiments, other embodiments, including embodiments which may or may not provide various features and advantages set forth herein will be apparent to those of ordinary skill in the art in view of the foregoing disclosure. The specific embodiments described above are disclosed as examples only.

### List of reference signs

- 1: garment
- 2: sensors
- 3: activators
- 4: controlling unit
- 5: wires
- 6: external device
- 7: external sensor
- 8: acoustic signal
- 9: voice output
- 10: receiving
- 11: processing
- 12: determining
- 13: stimulating
- 21: camera
- 22: display
- 23: processing unit
- 24: Internet

- 40: wireless module
- 41: EIM
- 42: EMG
- 43: BIA
- 45: EMS
- 50: microcontroller
- 51: stimulation power supply
- 52: current control
- 53: leading sign changer

## Claims

1. Garment (1) for training muscles of a user comprising:
sensors (2) embedded into the garment (1) for electrical contact with a skin surface of the user when the garment is worn;
activators (3) embedded into the garment to stimulate one or more muscles; and
a controlling unit (4) for receiving and processing signals representing an electrical impedance of one or more muscles;
wherein a stimulation of the one or more muscles through the activators (3) is generated when one or more muscles are in an un-exhausted state and no stimulation of the one or more muscles is generated when one or more muscles are in an exhausted state.

2. The garment of claim 1, wherein the sensors (2) and the controlling unit (4) are adapted to determine the electrical impedance representing a level of muscle fatigue by one or more of electrical impedance myography (EIM), electromyography (EMG), and a body composition with a process of bioelectrical impedance analysis (BIA).

3. The garment according to any preceding claim, wherein the activators (3) are laminated onto the garment.

4. The garment according to any preceding claim, wherein the activators (3) comprise electrodes (3) which are adapted to stimulate the one or more muscles by electronic muscle stimulation (EMS).

5. The garment according to any preceding claim, wherein the activators (3) stimulate the one or more muscles by a controlled current up to 30 mA.

6. The garment according to any preceding claim, wherein the controlling unit (4) is attached to the garment (1) and is adapted to communicate with an external device 6.

7. The garment according to any preceding claim, wherein the controlling unit (4) is adapted to connect to an external sensor (7).

8. The garment according to any preceding claim further comprising signaling means for generating feedback.

9. The garment according to claim 8, wherein the feedback is provided by vibration.

10. The garment according to claim 8 or 9, wherein the feedback is provided by an acoustic signal (8) communicating the beginning and end of an exercise and/or the feedback is provided by a voice output (9) communicating a correction of the form within an instructed movement.

11. A method for training muscles of a user wearing a garment (1) comprising the steps of:
receiving (10) signals representing an electrical impedance of one or more muscles;
processing (11) the signals to determine the electrical impedance;
determining (12) whether the one or more muscles are in an exhausted state; and
in response to the processed signals stimulating (13) the one or more muscles when one or more muscles are in an un-exhausted state and pausing the stimulation of the one or more muscles when one or more muscles are in an exhausted state.

12. The method of claim 11, wherein the step of processing (11) the signals further comprises determining the electrical impedance representing a level of muscle fatigue by one or more of electrical impedance myography (EIM), electromyography (EMG), and a body composition with a process of bioelectrical impedance analysis (BIA).

13. The method of claim 11 or 12, wherein measuring and stimulation of one or more muscles are performed substantially at the same time.

14. The method of any claim 11 to 13 further comprising the step of receiving (11) signals from an external sensor (5), the signals are indicative of one of: acceleration of a body or body parts, heart rate frequence, heart rate variability pulse, body-core temperature, and body hydration.

15. The method of any claim 11 to 14 further comprising the step of applying real-time visual motion analysis and in response to the analysis providing feedback.
